Europäisches Patentamt

European Patent Office

Office européen des brevets

(11) Publication number: **0 303 391 B1**

# EUROPEAN PATENT SPECIFICATION

(45) Date of publication of patent specification: **07.06.95**

(51) Int. Cl.6: **C07D 497/20, A61K 31/435,
//(C07D497/20,327:00,221:00,
221:00)**

(21) Application number: **88307162.3**

(22) Date of filing: **03.08.88**

(54) **Optical isomers of a spiroquinuclidine derivative.**

(30) Priority: **13.08.87 US 84799**

(43) Date of publication of application:
**15.02.89 Bulletin 89/07**

(45) Publication of the grant of the patent:
**07.06.95 Bulletin 95/23**

(84) Designated Contracting States:
**AT BE CH DE ES FR GB IT LI LU NL SE**

(56) References cited:

**"Cellular and Molecular Basis of Cholinergic
Function, edition 47, July 1987, pages
913-927, VHC, New York, US, edited by MJ.
Dowdall et al. A. FISHER et al.: "Restoration
of cognitive functions in an animal model of
Alzheimer's disease"**

(73) Proprietor: **State of Israel Represented by the
Prime Minister's Office The Israel Institute
for Biological Research
P.O. Box 19
Nes Ziona (IL)**

(72) Inventor: **Fisher, Abraham
47/7 David Elazar Street
Holon (IL)**
Inventor: **Karton, Ishai
37 Bialik Street
Ness-Ziona (IL)**

(74) Representative: **Holdcroft, James Gerald, Dr.
et al
Graham Watt & Co.,
Riverhead
Sevenoaks, Kent TN13 2BN (GB)**

EP 0 303 391 B1

**Description**

FIELD OF THE INVENTION

The present invention relates to the optical isomers of a spiroquinuclidine derivative, to pharmaceutical compositions comprising such isomers, to a method for treating diseases of the central nervous system therewith and to a process for preparing said isomers.

BACKGROUND OF THE INVENTION

The present applicants were co-inventors of previous patent applications relating to novel spiro-derivatives of quinuclidine, see e.g. European Patent Appln. No. 0205247 A2, published December 17, 1986, based on Israel Patent Applications Nos. 75166 filed May 10, 1985 and 77568 filed January 10, 1986, and commonly assigned U.S. Patent 4,855,290, the contents of which are incorporated herein by reference. These novel quinuclidine derivatives were found to possess central nervous system activity. The biological activity of the compound 2-methylspiro(1,3-oxathiolane-5',3)quinuclidine, which exists as geometrical cis- and trans-isomers depending upon whether the 2-methyl group is located on the same side of the oxathiolane ring as the quinuclidine ring nitrogen atom (cis) or on the other side of the quinuclidine ring nitrogen atom (trans), was in particular extensively investigated, and it was found on the basis of pre-clinical tests that the cis- compound could be especially promising for the control of senile dementia of Alzheimer's type (SDAT).

In addition to the existence of geometrical isomerism, the compounds in question also possess a potential for optical isomerism, as will be appreciated by those skilled in the art. As is also known, however, such a potential is not always readily realized in practice. Moreover, in view of the promise of the geometrical isomers of the compound 2-methylspiro(1,3-oxathiolane-5',3) quinuclidine for the treatment of SDAT, it is evidently of interest to know the relative biological activity of such compounds. The relationship between the direction of rotation of the plane of polarized light by a particular optical isomer on the one hand, and its biological potency on the other, is not predictable with any degree of certainty.

Therefore, it is a principal object of the invention to provide optical isomers of 2-methylspiro(1,3-oxathiolane-5',3)quinuclidine. Further objects of the invention, and especially those which relate to the provision of useful pharmaceutical compositions and methods for the treatment of disease in mammals, will be apparent from the description which follows.

SUMMARY OF THE INVENTION

The present invention is concerned with the individual optical isomers (+)-cis-, and (-)-cis-, 2-methylspiro(1,3-oxathiolan-5,3')quinuclidine, and especially such isomers in which the specific rotation has been determined. Preferably, such isomers are obtained in a highly pure state, more particularly having at least 95% optical purity.

The invention includes the acid addition salts of these individual optical isomers, and especially such addition salts which are pharmaceutically compatible.

Particular individual optical isomers provided in accordance with this aspect of the invention are the following:

(+)-cis-2-methylspiro(1,3-oxathiolan-5,3')quinuclidine, the free base in ethanol having $[\alpha]^{20}$ about + 32±1;

(-)-cis-2-methylspiro(1,3-oxathiolan-5,3')quinuclidine, the free base in ethanol having $[\alpha]^{20}$ about - 32±1.

The invention provides an optically active composition containing in admixture (a) the individual optical isomer (-)-cis-2-methylspiro(1,3-oxathiolan-5,3')quinuclidine, or its acid addition salt; and (b) the individual optical isomer (+)-cis-2-methylspiro(1,3-oxathiolan-5,3')quinuclidine, or its acid addition salt, with the proviso that the (±)-cis-racemate is excluded.

The invention also provides use for the manufacture of a medicament for treating diseases of the central nervous system in mammals, of an optically active composition according to the invention.

The invention also provides a pharmaceutical formulation for use in treating diseases of the central nervous system in mammals, which comprises optically active compositions according to the invention.

The pharmaceutical composition may also comprise an inert carrier or diluent. The composition may be, for example, in a form suitable for oral, rectal or parenteral administration, or for administration by insufflation or nasal spray. Alternatively, it may be in a form suitable for transdermal administration, and in this case it may comprise as an additional component, a low molecular weight fatty acid.

The expression "at least one member" or "at least one optical isomer", as used in the present specification defines one such member (i.e. optical isomer), and any mixture of two or more such members, except where such a mixture would constitute a racemate per se. These racemates are of course described and claimed in the prior patent applications which have been mentioned above, and corresponding granted patents.

The pharmaceutical composition may conveniently be in unit dosage form. Suitably, the at least one optical isomer referred to above, or a pharmaceutically compatible acid addition salt thereof, may be present in an amount in the range of about 0.5 to about 500 mg., together with an inert carrier or diluent. This amount lies preferably within the range of about 5 to about 100 mg., more preferably within the range of about 10 to about 50 mg.

The optical isomer (-)-cis-2-methylspiro(1,3-oxathiolan-5,3')quinuclidine appears to possess the highest desired biological potency of all the isomers. This (-)-cis-isomer may be used as the free base, or in the form of a pharmaceutically compatible acid addition salt thereof, and the pharmaceutical compositions which contain this isomer preferably comprise additionally (for reasons which will become clearer infra) at least one compound selected from the group consisting of physostigmine, tetrahydroaminoacridine, choline, lecithin, piracetam, aniracetam, pramiracetam, oxiracetam, 4-aminopyridine, 3,4-diaminopyridine, somatostatin, pirenzepine, N-methylatropine, N-butylscopolamine, scopolamine, clonidine, quanfamicine or Nerve Growth Factor, and desirably also, an inert carrier or diluent.

The invention further provides a pharmaceutical composition provided in two part form, a part A comprising the optical isomers of the invention and a part B comprising an additional compound selected from those recited above.

The invention also provides according to still a further aspect, a process for the preparation of at least one of the individual optical isomers described herein, which comprises treating a base selected from the group consisting of (±)-cis-2-methylspiro(1,3-oxathiolan-5,3')quinuclidine and (±)-trans-2-methylspiro(1,3-oxathiolan-5,3')quinuclidine with an amount of enantiomeric acid insufficient for stoichiometric reaction, and fractionating the precipitated salt until a fraction of desired optical purity is obtained. The mother liquors and the precipitate may be further processed as described below.

DETAILED DESCRIPTION OF THE INVENTION

The present inventors have found that when attempts are made to resolve either the cis- or the trans-2-methylspiro(1,3-oxathiolan-5,3')quinuclidine, by fractional crystallization using d- and l-tartaric acid, then in each case the respective enantiomers (+) and (-) are obtained. The total of four isomers exhausts all the possibilities, as may be seen from a consideration of the structural formulae in question [see, for example, Saunders et al, J. Med. Chem. 30: 969-75 (1987), the contents of which article are incorporated herein by reference, and in which article dioxolan analogues of the present compounds were prepared by a synthetic route followed by chromatographic separation, and not by optical resolution of racemates as in the present invention].

According to the optical resolution process of the present invention, (±)-cis-2-methylspiro(1,3-oxathiolan-5,3')quinuclidine is treated with an amount of enantiomeric acid insufficient for stoichiometric reaction, and the precipitated salt is fractionated until a fraction of desired optical purity is obtained. The enantiomeric acid may be e.g. L- or D-tartaric acid, or/and L- or D-mandelic acid, although other optically active acids may be used for the same purpose, as of course is well known in the art. The amount of enantiomeric acid may be e.g. about 50% of the equimolar amount which would be necessary to react with all the racemic base present. The reaction may be carried out in an organic solvent such as ethanol. As is also known, it is within the competence of one skilled in the art to select a suitable solvent for the purpose. The mode of fractionation is conveniently fractional crystallization. The present inventors have found that a small number of crystallizations (e.g. two) will normally give a product of high optical purity, e.g. 95% or better.

The mother liquor or liquors from the salt forming reaction and/or recrystallization may be basified and the residual base may then be treated with the enantiomer of the enantiomeric acid originally used, and the precipitated salt is again fractionated until a fraction of desired optical purity is obtained. This stage will give a salt of the base, wherein the latter has the opposite optical rotation of the base present in the salt obtained in the first stage of the salt forming reaction.

In order to obtain the desired enantiomeric bases, either or both salts formed in the first and second stages just described may be treated with a base such as an alkali metal hydroxide. There will thus be obtained an enantiomeric base selected from (+)- and (-)-cis-2-methylspiro(1,3-oxathiolan-5,3')quinuclidine. Such an enantiomeric base may of course be treated with an acid (especially a pharmaceutically compatible acid) to form an acid addition salt thereof. Examples of suitable such acids are described below.

The individual optical isomers of the present invention are muscarinic agonists with a high specificity for the central nervous system. They are highly specific for a subpopulation of muscarinic receptors, namely pirenzepine-sensitive M1 receptors. Due to their pharmacological properties, these compounds can activate central cholinergic functions under conditions where the cholinergic system is hypofunctional. These compounds [and especially the (-)-cis-isomer] can therefore be utilized for the treatment of conditions which as presenile dementia, senile dementia of Alzheimer's type (SDAT), mixed Alzheimer's and Parkinson's disease, tardive dyskinesia, acute confusion conditions, hyperkinesia, mania, Pick's disease, Huntington's chorea, Friedrich's ataxia, Down's syndrome, Gilles de la Tourette disease, post encephalitic amnesic syndrome, alcohol withdrawal symptons, and progressive supranuclear palsy, because all of these disease states are disturbances in which a central cholinergic hypofunction has been implicated at least to a certain extent.

These compounds [and especially the (-)-cis-isomer] would appear to be of value for the treatment of SDAT. Thus, in SDAT patients, the (-)-cis-isomer in particular can be used in combination with anticholinesterase inhibitors such as physostigmine or tetrahydroaminoacridine; in combination with acetylcholine precursors such as choline or lecithin; in addition to "nootropic" drugs such as piracetam, aniracetam, oxiracetam or pramiracetam; in addition to compounds that interact with $Ca^{2+}$ channels such as 4-aminopyridine or 3,4-diaminopyridine; or in addition to peptides that can have modulatory effects on acetylcholine release, such as somatostatin; in combination with a peripheral antimuscarinic agent (such as pirenzepine, N-methylatropine or N-butylscopolamine) to counteract peripheral adverse effects that might be expected at high doses, such as salivation, diarrhea, gastric secretion or vomiting, or in combination with transdermal scopolamine such as Scopoderm[R] to counteract nausea and/or vomiting; in combination with an adrenergic agonist (clonidine or quanfamicine) in order to alleviate both the cognitive and other impairments associated with a mixed cholinergic-noradrenergic deficiency in SDAT; in combination with Nerve Growth Factor (NGF, which is administered either by a nasal spray or intracerebroventicularly).

The compositions of the present invention, with or without the aforementioned other active substances, can be administered for example, by way of injection in a suitable diluent or carrier, per os, rectally in the form of suppositories, by way of insufflation or nasal spray, by infusion or transdermally in a suitable vehicle with or without physostigmine, for example by using the device which is the subject of Israel Patent Application No. 72694 (vide infra). This compound may also be used in disturbances where cholinergic underactivity is induced by drugs.

The (-)-cis-isomer, in admixture with the (+)-cis isomer is also of use for the treatment of disorders requiring the application of a long-lasting cholinergic agent of mild local activity. Such an agent is needed in disorders such as glaucoma, as the compound is not destroyed by the enzyme which deactivates acetylcholine, i.e. acetyl- and butyryl-cholinesterase. This compound may also be used for the treatment of peripheral cholinergic disorders such as myasthenia gravis, urinary bladder dysfunctions, Adi's disease and Eaton-Lambret disease.

The individual optical isomers of the present invention form stable addition salts with organic and inorganic acids. While for therapeutic purposes such salts should be pharmaceutically compatible, nevertheless it may be convenient, as for example for the purpose of isolation, to employ acid addition salts which are not pharmaceutically compatible, and the invention includes also the latter kind of acid addition salts. As will be obvious to those skilled in the art, the free bases may be converted to acid addition salts by reaction with the appropriate acid, and the salts may be converted by reaction with a base (e.g., an alkali metal hydroxide in aqueous solution) to the corresponding free bases.

The term "pharmaceutically compatible acid addition salt" as used herein refers to a combination of said quinuclidine derivative with relatively non-toxic inorganic or organic acids. Illustrative only of suitable acids are sulfuric, phosphoric, hydrochloric, hydrobromic, hydriodic, sulfamic, methanesulfonic, benzenesulfonic, para-toluenesulfonic, acetic, lactic, succinic, maleic, tartaric, citric, gluconic, ascorbic, benzoic and cinnamic acids.

Where the term "pharmaceutical composition" is used in the present specification and claims, this is to be understood in the sense that it may be suitable for human and/or veterinary treatment. In the pharmaceutical compositions of the invention, suitable pharmaceutical carriers and diluents, which comprise both solids and liquids, may, by way of example only, be selected from corn starch, lactose, calcium phosphate, stearic acid, polyethylene glycol, water, sesame seed oil, peanut oil, or propylene glycol. This composition may be in a form suitable for oral, rectal or parenteral administration, or for administration by insufflation or nasal spray, or in particular it may be in a form suitable for transdermal administration, and in any event the composition may be in unit dosage form. Exemplary compositions may take the for: of tablets, powder, granules, capsules, suspensions, solutions, suppositories, elixirs or ointments.

When the pharmaceutical composition is to be administered transdermally, it is preferred to utilize the drug delivery system according to Israel Patent Application No. 72684, although transdermal administration in accordance with the invention is not of course limited to this system. Thus, as mentioned previously, the pharmaceutical compositions of the invention adapted for transdermal administration may comprise as an additional ingredient, a low molecular weight fatty acid.

For the reasons noted hereinbefore, such a composition may contain as a further ingredient or ingredients, one or more compounds selected from the group consisting of physostigmine, tetrahydroaminoacridine, choline, lecithin, piracetam, aniracetam, pramiracetam, oxiracetam, 4-aminopyridine, 3,4-diaminopyridine, somatostatin, pirenzepine, N-methylatropine, N-butylscopolamine, scopolamine, clonidine, quanfamicine or Nerve Growth Factor.

As has already been indicated, the present invention provides a method for treating diseases of the central nervous system in mammals (especially diseases due to a deficiency in the central cholinergic system), which comprises administering to the mammal, preferably in the form of a pharmaceutical composition as described above, a composition comprising of (+)-cis, and (-)-cis -2-methylspiro(1,3-oxathiolan-5,3')quinuclidine, or pharmaceutically compatible acid addition salts thereof. According to an embodiment of this aspect of the invention, a method for treating senile dementia of Alzheimer's type comprises administering to a patient a pharmaceutical composition, as described above. For this purpose, the composition may be administered via the oral route in an amount which lies within the range of about 0.1 to about 60 mg./kg., preferably about 0.5 to about 10 mg./kg. body weight, more preferably about 1 to about 5 mg./kg. body weight. within the range of about 1 to about 5 mg./kg. body weight. On the other hand, this composition may be administered via the parenteral route (which includes, for example, intramuscular, intravenous and subcutaneous administration) in an amount which lies within the range of about 0.01 to about 40 mg./kg., preferably about 0.05 to about 5 mg./kg. body weight, more preferably about 0.1 to about 2 mg./kg. body weight.

In prescribing a particular form and rate of administration, the physician will of course take into consideration the usual factors such as the severity of the symptoms, the physical circumstances of the patient, and so forth.

Taking into account the usual weight ranges of patients, the foregoing dosage ranges, and the possibility that it may be desirable to administer multiple rather than single doses, pharmaceutical compositions in accordance with the invention which are adapted for oral or parenteral administration, may contain the optically active ingredients, in an amount (for example) in the range of about 0.5 to about 500 mg., preferably about 5 to about 100 mg., more preferably in the range of about 10 to about 50 mg.

For the purpose of definition, it is intended that the expression "method for the treatment of diseases of the central nervous system", and like expressions, throughout the specification and claims, be taken to include a method for the prevention of drug-induced diseases of the central nervous system.

EXAMPLE I

Separation of the individual enantiomers of cis- and trans-2-methylspiro(1,3-oxathiolan-5,3')quinuclidine.

Cis-2-methylspiro(1,3-oxathiolan-5,3')quinuclidine as the free base (37,5 g., 0.108 mole) and L-tartaric acid (14.08 g., 0.094 mole) were placed in a 2 liter flask, absolute ethanol (1120 g.) was added, the mixture was refluxed for 10 minutes and then allowed to stand overnight. The resulting precipitate was filtered off and recrystallized from absolute ethanol. The solid product was basified and reprecipitated as the hydrochloride salt (yield: 11.7 g., 26%); it had m.p. 198-200°C and $[\alpha]^{20}$ + 32±1 (free base in ethanol).

The mother liquor was evaporated, basified and treated with D-tartaric acid in the same manner. After two recrystallizations, the resulting tartrate was basified and reprecipitated as the hydrochloride salt (yield: 14.7 g., 33.2%); it had m.p. 198-200°C and $[\alpha]^{20}$ - 32±1 (free base in ethanol).

The following properties apply to each enantiomer. $R_f$ (TLC, neutral alumina, chloroform) 0.7. NMR spectrum using pure 2,2,2-trifluoro-1-(9-anthryl)ethanol ($C_6D_6$) shows the presence of a single enantiomer (> 95% optical purity). The NMR spectrum of the hydrochloride salt in $CDCl_3$ is identical to that of the (±)-cis-isomer hydrochloride.

By a similar procedure to the above there were obtained:

(-)-trans-2-methylspiro(1,3-oxathiolan-5,3')quinuclidine, the free base in ethanol having $[\alpha]^{20}$ - 24±1.2 and

(+)-trans-2-methylspiro(1,3-oxathiolan-5,3')quinuclidine, the free base in ethanol having $[\alpha]^{20}$ + 24±1.2.

In the latter instance, there was used additionally l-mandelic acid and the l-mandelate was recrystallized from ethyl acetate.

EXAMPLE II

Biological Testing of the Isolated Enantiomers

The LD$_{50}$ in mice of (+)-, (-) and [for comparison] (±)-cis-2-methylspiro(1,3-oxathiolan-5,3')quinuclidine (HCl salts) is shown in Table 1. There are no significant differences in the toxicity of the tested compounds.

Table 1

|  |  | LD$_{50}$ (mg./kg.) | 95% confidence limits |
|---|---|---|---|
| (±)-isomer | male | 45 | 40.9 - 49.6 |
|  | female | 40.8 | 36.6 - 45 |
| (+)-isomer | male | 39.1 | 35.6 - 42.6 |
|  | female | 45.0 | 33.8 - 56.3 |
| (-)-isomer | male | 35 | 26.3 - 43.8 |
|  | female | 40 | 30 - 50 |

The potency of putative cholinergic compounds in displacing from rat forebrain homogenates the following $^3$H-labelled compounds, namely, $^3$H-quinuclidinyl benzilate ($^3$H-QNB; a non-selective M$_1$ and M$_2$ antagonist), $^3$H-Pirenzepine ($^3$H-PZ; a selective M$_1$ antagonist) and $^3$H-cis-dioxolane($^3$H-CD, a non-selective M$_1$ and M$_2$ agonist), is shown in Table 2. Included in this study for comparative purposes are Oxotremorine (mainly an M$_2$ agonist), McN-A-343 (mainly an M$_1$ agonist), and (±)-cis- & trans-2-methylspiro(1,3-oxathiolan-5,3')quinuclidine.

## Table 2

| Compd. tested # | (a) Ki($^3$H-PZ) µM | (b) Ki($^3$H-QNB) µM | (c) Ki($^3$H-CD) µM | (d) a:b | (e) a:c |
|---|---|---|---|---|---|
| 1 | 0.6 ±0.2 (4) | 1.7 ±0.3 (8) | 9.6 ±4.2 (2)* | 0.35 | 625 |
| 2 | 4.9 ±1.9 (4) | 14.2 ±1.41(3) | 0.4 ±0.1 (4) | 0.35 | 12.25 |
| 3 | 14.6 ±2.9 (4) | 24.7 ±2.3 (8) | 1.8 ±0.1 (2) | 0.59 | 8.1 |
| 4 | 0.6 ±0.15(7) | 6.3 ±1.0 (15) | 0.45±0.18(4) | 0.10 | 1.4 |
| 5 | 1.1 ±0.3 (3) | 3.5 ±1.5 (3) | 0.21±0.06(2) | 0.32 | 5.2 |
| 6 | 9.6 ±1.8 (3) | 36.4 ±8 (3) | 7.5 (1) | 0.26 | 1.3 |

Key to Table 2:

$$Ki = IC_{50} \div (1 + C/K_D),$$ where C is the concentration of the radioactive ligand and $K_D$ is the dissociation constant thereof.

compounds tested –

#1 = Oxotremorine (*expressed in M x 10$^{-10}$)
#2 = McN-A-343
#3 = (±)-trans-isomer
#4 = (±)-cis-isomer
#5 = (-)-cis-isomer
#6 = (+)-cis-isomer

Discussion of Table 2

From Table 2, it is evident that the (±)-cis-isomer has the highest $M_1$ selectivity followed by its (-) and (+) enantiomers. The (-)-cis-enantiomer is 2.2 times more potent in $^3$H-QNB displacement than its racemate. Moreover, the (±)-cis-isomer is the most selective $M_1$ agonist, being more selective than the prototype $M_1$ agonist McN-A-343. As can be seen from the ratios (Ki($^3$H-PZ):Ki($^3$H-CD) and Ki($^3$H-PZ):Ki($^3$H-QNB) in columns (d) and (e) of the Table, the structurally rigid compounds of the present invention showed higher selectivity towards $M_1$ receptors than McN-A-343 or Oxotremorine. However, there were some apparent discrepancies between the order of the ratios Ki($^3$H-PZ):Ki($^3$H-CD) and Ki($^3$H-PZ):Ki($^3$H-QNB) among the tested compounds, especially in regard to Oxotremorine, the (±)-trans-isomer and McN-A-343. While the order of the ratios Ki($^3$H-PZ):Ki($^3$H-CD) for these three compounds was oxotremorine > McN-A-343 > (±)-trans-isomer, the order of the ratios Ki($^3$H-PZ):Ki($^3$H-QNB) was McN-A-343 > (±)-trans-isomer > oxotremorine. These ratios are indices for the relative selectivity of the tested compounds towards $M_1$ as against $M_2$ receptors. It is not clear why in one test, oxotremorine showed the weakest relative affinity towards $M_1$ binding sites [Ki($^3$H-PZ):Ki($^3$H-CD)] whereas in the other test its relative affinity towards the $M_1$ binding sites was relatively stronger than McN-A-343 and the (±)-trans-isomer [Ki($^3$H-PZ):Ki($^3$H-QNB)]. One explanation of this phenomenon is that since the affinity of oxotremorine towards all muscarinic receptors is greater than those of the weaker agonists McN-A-343 or the (±)-trans-isomer [Watson et al, JPET 237: 411-418 (1986)], then lower concentrations of oxotremorine are needed to displace both $^3$H-PZ and $^3$H-QNB from forebrain homogenate, especially $^3$H-PZ which possesses lower affinity towards muscarinic receptors than $^3$H-QNB. Thus, in principle, strong agonists will yield a lower Ki($^3$H-PZ):Ki($^3$H-QNB) ratio than weak agonists, without taking into consideration the selectivity of the selected ligands. On the other hand, the $K_D$ of $^3$H-PZ similar to that of $^3$H-CD and therefore weak and strong agonists should behave similarly in displacing these labelled ligands, unless their selectivity towards one receptor subpopulation is different. Therefore, the ratio Ki($^3$H-PZ):Ki($^3$H-CD) should represent more accurately the relative affinities towards $M_1$ receptors. Indeed, the order of these ratios for the compounds tested is consistent with the order found for the ratios $IC_{50}$(FB):$IC_{50}$(CER) which also serves as an index for $M_1$ versus $M_2$ selectivity, namely (±)-cis-isomer > (±)-trans-isomer > oxotremorine (FB = forebrain, which contains mainly $M_1$ receptors; CER = cerebellum, which contains mainly $M_2$ receptors).

Table 3 shows the results of a study of isolated guinea pig ileum induced contraction. From this Table also, it is evident that the (-)-cis-isomer of the present invention is twice as active as the corresponding racemate, while the (+)-cis-isomer lacks significant agonist activity.

Table 3

| Compound | EC50 (M) | type of activity |
|---|---|---|
| Acetylcholine | $5 \times 10^{-8}$ | agonist |
| (±)-cis-isomer | $3 \times 10^{-6}$ | agonist |
| (-)-cis-isomer | $1.5 \times 10^{-6}$ | agonist |
| (+)-cis-isomer | $> 10^{-5}$ | inactive as agonist |
| (±)-trans-isomer | $> 10\text{-}5$ | agonist |

## BEHAVIORAL EXPERIMENTS

Ethylcholine aziridinium ion (hereinafter denoted AF64A) is a selective presynaptic cholinergic neurotoxin, which on intracerebroventricular injection in rats induces persistent cholinergic hypofunction that mimics the cortical and hippocampal cholinegic deficiency and the cognitive impairments reported in SDAT. Accordingly, the possibility of reversal of the AF64A-induced effects by (-)- and [for comparison] (±)-cis-2-methylspiro(1,3-oxathiolan-5,3')quinuclidine in the passive avoidance task was studied. In this experiment, the results of which are reported in Table 4, we investigated the effect of these substances (0.2mg./kg/., per os) on the retention after 72 hours of an inhibitory learning (passive avoidance-step through) task in AF64A and saline-injected rats, using a post-training drug treatment paradigm. The surgery carried out on the test animals (Sprague-Dawley rats raised by Charles River) was similar to that described in our prior published European Patent Application No. 0205247. The behavioral studies were performed one month after surgery.

## Behavioral Testing

The behavioral testing procedure comprised two phases, 1 and 2. In a pretest training procedure each rat was individually placed in a small lighted front compartment of a two-compartment box. After a 60 secs. familiarization/adaptation period, the door separating the two compartments was opened and a clock activated. The rat's latency to enter the large dark compartment of the box (to step-through) was measured. Immediately following entry into the dark compartment, the rat was subjected to an inescapable scrambled foot shock applied to the grid floor (0.6mA for 3 secs.). Sixty secs. after the termination of the shock, at the end of the training procedure, the rat was removed from the dark compartment and double distilled water, (-)- and (±)-cis-2-methylspiro(1,3-oxathiolan-5,3')quinuclidine were administered per os. Rats were then returned to their home cage. Retention of the passive avoidance task was measured 24 hr. after training by again placing the rat in the lighted front compartment, and after a 60 secs. adaptation period, measuring the latency to enter the dark compartment. The test session ended when a rat entered the dark compartment or after 600 secs. had elapsed. Animals that failed to step through within 600 secs. were removed from the apparatus and a 600 secs. latency was recorded for them. The results are summarized in Table 4.

EP 0 303 391 B1

Table 4

| Retention latency after 72 hours (in seconds) | | | |
|---|---|---|---|
| Rats | double-distilled water | (±)-cis-isomer | (-)-cis-isomer |
| AF64A-treated | 181.4 (±53.3) | 407.9 (±72.1) | 563.5 (±23.6) |
| saline-treated | 593.3 (±47.5) | 478.2 (±65.7) | 474 (±61.3) |
| Note: nine rats were treated in each group. | | | |

Discussion

The net latencies of AF64A-treated groups, subsequently treated with either (-)- or (±)-cis-2-methylspiro-(1,3-oxathiolan-5,3')quinuclidine were significantly longer ($p < 0.005$ and $p < 0.05$, respectively) than the group subsequently treated with double distilled water. Therefore, the retention of the step-through passive avoidance response of the AF64A-injected groups was significantly improved by both compounds, while the saline-treated groups did not change significantly when subjected to this subsequent treatment. This result provided an indication that administration of the (-)- or the (±)-cis-isomers can counter the cognitive impairment induced by the cholinergic neurotoxin AF64A.

While certain embodiments of the invention have been particularly described, it will be apparent to those skilled in the art that many modifications and variations may be made. The invention is accordingly not to be construed as restricted to such embodiments, rather its scope will be defined in accordance with the following claims.

In appropriate cases compositions according to the invention can be provided in two-part form.

**Claims**
**Claims for the following Contracting States : DE, GB, FR, IT, NL, SE, LI, CH, BE, AT, LU**

1.  An optically active composition containing in admixture (a) the individual optical isomer (-)-cis-2-methylspiro(1,3-oxathiolan-5,3')quinuclidine, or its acid addition salt; and (b) the individual optical isomer (+)-cis-2-methylspiro(1,3-oxathiolan-5,3')quinuclidine, or its acid addition salt, with the proviso that the (±)-cis-racemate is excluded.

2.  An optically active composition according to claim 1, for use in therapy for example in the treatment of diseases of the central nervous system in mammals.

3.  Use for the manufacture of a medicament for treating diseases of the central nervous system in mammals, of an optically active composition according to claim 1.

4.  A pharmaceutical formulation (e.g. in unit dosage form) for use in treating diseases of the central nervous system in mammals, which comprises an optically active composition according to claim 1.

5.  A use or formulation according to claim 3 or claim 4, wherein said composition contains at least one additional ingredient (and is e.g. in two part form), the additional ingredient being selected from anticholinesterase inhibitors (e.g. physostigmine, tetrahydroaminoacridine), acetylcholine precursors (e.g. choline, lecithin), nootropic drugs (e.g. piracetam, aniracetam, pramiracetam, oxiracetam), compounds that react with Ca + + channels (e.g. 4-aminopyridine, 3,4-diaminopyridine), peptides that can have modulatory effects on acetylcholine release (e.g. somatostatin), peripheral antimuscarinic agents (e.g. pirenzepine, N-methylatropine, N-butylscopolamine), scopolamine (e.g. transdermal scopolamine), andrenergic agonists (e.g. clonidine or quanfamicine), and Nerve Growth Factor.

6.  A use or formulation according to any one of claims 3, 4 and 5, wherein said composition contains additionally at least one inert carrier or diluent.

7.  A pharmaceutical formulation according to any one of claims 4, 5 and 6, which is in a form suitable for oral, rectal, parenteral or transdermal administration, or for administration by insufflation or nasal spray.

9

8. A pharmaceutical formulation according to claim 7, which is in a form suitable for transdermal administration and which comprises as an additional component, a low molecular weight fatty acid.

9. A pharmaceutical formulation according to any one of claims 4 to 8 in unit dosage form, wherein said optically active composition is present in an amount in the range of 0.5 to 500 mg, for example 5 to 100 mg, e.g. 10 to 50 mg, per dosage unit.

10. A composition, use or formulation according to any of claims 2 to 9, wherein the disease is due to a deficiency in the central cholinergic system.

11. A composition or formulation use according to any one of the claims 2 to 9, wherein the disease is senile dementia of Alzheimer's type.

12. A composition, use or formulation according to claim 11, wherein administration is:
  (i) via the oral route in an amount of the optically active composition which lies within the range of 0.1 to 60 mg/kg; for example 0.5 to 10 mg/kg, e.g. 1 to 5 mg/kg body weight; or
  (ii) via the parenteral route in an amount of the optically active composition which lies within the range of 0.01 to 40 mg/kg; for example 0.05 to 5 mg/kg, e.g. 0.1 to 2 mg/kg body weight.

**Claims for the following Contracting State : ES**

1. A method for preparing an optically active composition containing in admixture (a) the individual optical isomer (-)-cis-2-methylspiro(1,3-oxathiolan-5,3')quinuclidine, or its acid addition salt; and (b) the individual optical isomer (+)-cis-2-methylspiro(1,3-oxathiolan-5,3')quinuclidine, or its acid addition salt, with the proviso that the (±)-cis-racemate is excluded, by either mixing (a) and (b) together, or by partial optical resolution of (±)-cis,racemate.

2. A method according to claim 1, wherein the optically active composition is for use in therapy, for example in the treatment of diseases of the central nervous system in mammals.

3. Use for the manufacture of a medicament for treating diseases of the central nervous system in mammals, of an optically active composition as defined in claim 1.

4. A method of producing a pharmaceutical formulation (e.g. in unit dosage form) for use in treating diseases of the central nervous system in mammals, by incorporating in the formulation an optically active composition as defined in claim 1.

5. A use or method according to claim 3 or claim 4, wherein said composition contains at least one additional ingredient (and is e.g. in two part form), the additional ingredient being selected from anticholinesterase inhibitors (e.g. physostigmine, tetrahydroaminoacridine), acetylcholine precursors (e.g. choline, lecithin), nootropic drugs (e.g. piracetam, aniracetam, pramiracetam, oxiracetam), compounds that react with Ca++ channels (e.g. 4-aminopyridine, 3,4-diaminopyridine), peptides that can have modulatory effects on acetylcholine release (e.g. somatostatin), peripheral antimuscarinic agents (e.g. pirenzepine, N-methylatropine, N-butylscopolamine), scopolamine (e.g. transdermal scopolamine), andrenergic agonists (e.g. clonidine or quanfamicine), and Nerve Growth Factor.

6. A use or method according to any one of claims 3, 4 and 5, wherein said composition contains additionally at least one inert carrier or diluent.

7. A method according to any one of claims 4, 5 and 6, wherein the pharmaceutical formulation is in a form suitable for oral, rectal, parenteral or transdermal administration, or for administration by insufflation or nasal spray.

8. A method according to claim 7, wherein the pharmaceutical formulation is in a form suitable for transdermal administration and which comprises as an additional component, a low molecular weight fatty acid.

9. A method according to any one of claims 4 to 8, wherein the pharmaceutical formulation is in unit dosage form, and said optically active composition is present in an amount in the range of 0.5 to 500 mg, for example 5 to 100 mg, e.g. 10 to 50 mg, per dosage unit.

10. A method or use according to any one of claims 2 to 9, wherein the disease is due to a deficiency in the central cholinergic system.

11. A method or use according to any one of the claims 2 to 9, wherein the disease is senile dementia of Alzheimer's type.

12. A method or use according to claim 11, wherein administration is:
    (i) via the oral route in an amount of the optically active composition which lies within the range of 0.1 to 60 mg/kg; for example 0.5 to 10 mg/kg, e.g. 1 to 5 mg/kg body weight; or
    (ii) via the parenteral route in an amount of the optically active composition which lies within the range of 0.01 to 40 mg/kg; for example 0.05 to 5 mg/kg, e.g. 0.1 to 2 mg/kg body weight.

**Patentansprüche**
**Patentansprüche für folgende Vertragsstaaten : DE, GB, FR, IT, NL, SE, LI, CH, BE, AT, LU**

1. Optisch aktive Zusammensetzung, die in einer Mischung (a) das einzeln optische Isomer (-)-cis-2-methylspiro(1,3-oxathiolan-5,3')chinuclidin oder sein durch Säurezugabe erhaltenes Salz; und (b) das einzeln optische Isomer (+)-cis-2-Methylspiro(1,3-oxathiolan-5,3')chinuclidin oder sein durch Säurezugabe erhaltenes Salz, mit dem Vorbehalt, daß das (±)-cis-Racemat ausgeschlossen ist, enthält.

2. Optisch aktive Zusammensetzung nach Anspruch 1, die in der Therapie, zum Beispiel bei der Behandlung von Krankheiten des zentralen Nervensystems bei Säugetieren, verwendet wird.

3. Verwendung zur Herstellung eines Medikamentes mit einer optisch aktiven Zusammensetzung nach Anspruch 1 zur Behandlung von Krankheiten des zentralen Nervensystems bei Säugetieren.

4. Pharmazeutische Formulation (z.B. in Grunddosierungsform) zur Verwendung bei der Behandlung von Krankheiten des zentralen Nervensystems bei Säugetieren, die eine optisch aktive Zusammensetzung nach Anspruch 1 umfaßt.

5. Verwendung oder Formulation nach Anspruch 3 oder 4, bei denen die Zusammensetzung wenigstens einen zusätzlichen Bestandteil enthält und z.B. in zweiteiliger Form vorliegt, der zusätzliche Bestandteil wird aus Anticholinesteraseinhibitoren (z.B. Physiostigmin, Tetrahydroaminoacridin), Acetylcholinvorstufen (z.B. Cholin, Lecithin), nootrope Arzneimitteln (z.B. Piracetam, Aniracetam, Pramiracetam, Oxiracetam), Verbindungen, die mit dem $Ca^{++}$-Kanal reagieren (z.B. 4-Aminopyridin, 3,4-Diaminopyridin), Peptiden, die modulatorische Wirkungen auf die Freisetzung von Acetylcholin aufweisen können (z. B. Somatostatin), peripheren Antimuskarinmitteln (z.B. Pirenzepin, N-Methylatropin, N-Butylscopolamin), Scopolamin (z.B. transdermales Scopolamin), adrenergen Agonisten (z.B. Clonidin oder Quanfamicin) und Nervenwachstumsfaktor ausgewählt.

6. Verwendung oder Formulation nach einem der Ansprüche 3, 4 und 5, bei der die Zusammensetzung zusätzlich wenigstens ein inertes Träger- oder Verdünnungsmittel enthält.

7. Pharmazeutische Formulation nach einem der Ansprüche 4, 5 und 6, die in einer geeigneten Form zur oralen, rektalen, parenteralen oder transdermalen Administration oder zur Administration durch Einblasen oder Nasenspray vorliegt.

8. Pharmazeutische Formulation nach Anspruch 7, die in einer geeigneten Form zur transdermalen Administration vorliegt und die als eine zusätzliche Komponente eine Fettsäure mit niedrigem Molekulargewicht umfaßt.

9. Pharmazeutische Formulation nach einem der Ansprüche 4 bis 8 in einer Grunddosierungsform, bei der die optisch aktive Zusammensetzung in einer Menge im Bereich von 0,5 bis 500 mg, zum Beispiel 5 bis 100 mg, z.B. 10 bis 50 mg, pro Dosierungseinheit, vorhanden ist.

**10.** Zusammensetzung, Verwendung oder Formulierung nach einem der Ansprüche 2 bis 9, bei der die Krankheit eine Folge eines Fehlers des zentralen cholinergen Systems ist.

**11.** Zusammensetzung oder Formulationsverwendung nach einem der Ansprüche 2 bis 9, bei der die Krankheit Altersdemenz vom Alzheimertyp ist.

**12.** Zusammensetzung, Verwendung oder Formulation nach Anspruch 11, bei denen die Adminstration:
(i) über den oralen Weg in einer Menge der optisch aktiven Zusammensetzung, die im Bereich von 0,1 bis 60 mg/kg; zum Beispiel 0,5 bis 10 mg/kg, z.B. 1 bis 5 mg/kg Körpergewicht, liegt; oder
(b) über den parenteralen Weg in einer Menge der optisch aktiven Zusammensetzung, die im Bereich von 0,01 bis 40 mg/kg; zum Beispiel 0,05 bis 5 mg/kg, z.B. 0,1 bis 2 mg/kg Körpergewicht liegt,
erfolgt.

**Patentansprüche für folgenden Vertragsstaat : ES**

**1.** Verfahren zur Herstellung einer optisch aktiven Zusammensetzung, die in einer Mischung (a) das einzeln optische Isomer (-)-cis-2-methylspiro(1,3-oxathiolan-5,3')chinuclidin oder sein durch Säurezugabe erhaltenes Salz; und (b) das einzeln optische Isomer (+)-cis-2-Methylspiro(1,3-oxathiolan-5,3')-chinuclidin oder sein durch Säurezugabe erhaltenes Salz, mit dem Vorbehalt, daR das (±)-cis-Racemat ausgeschlossen ist, enthält, entweder durch Zusammenmischen von (a) und (b) oder durch teilweise optische Aufspaltung des (±)-cis-Racemats.

**2.** Verfahren nach Anspruch 1, bei dem die optisch aktive Zusammensetzung in der Therapie, zum Beispiel bei der Behandlung von Krankheiten des zentralen Nervensystems bei Säugetieren, verwendet wird.

**3.** Verwendung zur Herstellung eines Medikamentes einer optisch aktiven Zusammensetzung nach Anspruch 1 zur Behandlung von Krankheiten des zentralen Nervensystems bei Säugetieren.

**4.** Verfahren zur Herstellung einer pharmazeutischen Formulation (z.B. in Grunddosierungsform), die zur Behandlung von Krankheiten des zentralen Nervensystems bei Säugetieren durch Einbringen einer optisch aktiven Zusammensetzung in die Formulation nach Anspruch 1, verwendet wird.

**5.** Verwendung oder Verfahren nach Anspruch 3 oder 4, bei denen die Zusammensetzung wenigstens einen zusätzlichen Bestandteil enthält und z.B. in zweiteiliger Form vorliegt, der zusätzliche Bestandteil wird aus Anticholinesteraseinhibitoren (z.B. Physiostigmin, Tetrahydroaminoacridin), Acetylcholinvorstufen (z.B. Cholin, Lecithin), nootrope Arzneimittel (z.B. Piracetam, Aniracetam, Pramiracetam, Oxiracetam), Verbindungen, die mit dem $Ca^{++}$-Kanal reagieren (z.B. 4-Aminopyridin, 3,4-Diaminopyridin), Peptiden, die modulatorische Wirkungen auf die Freisetzung von Acetylcholin aufweisen können (z.B. Somatostatin), peripheren Antimuskarinmitteln (z.B. Pirenzepin, N-Methylatropin, N-Butylscopolamin), Scopolamin (z.B. transdermales Scopolamin), adrenergen Agonisten (z.B. Clonidin oder Quanfamicin) und Nervenwachstumsfaktor ausgewählt.

**6.** Verwendung oder Verfahren nach einem der Ansprüche 3, 4 und 5, bei der die Zusammensetzung zusätzlich wenigstens ein inertes Träger- oder Verdünnungsmittel enthält.

**7.** Verfahren nach einem der Ansprüche 4, 5 und 6, bei dem die pharmazeutische Formulation in einer geeigneten Form zur oralen, rektalen, parenteralen oder transdermalen Administration oder zur Administration durch Einblasen oder Nasenspray vorliegt.

**8.** Verfahren nach Anspruch 7, bei dem die pharmazeutische Formulation in einer geeigneten Form zur transdermalen Administration vorliegt und die als eine zusätzliche Komponente eine Fettsaure mit niedrigem Molekulargewicht umfaßt.

**9.** Verfahren nach einem der Ansprüche 4 bis 8, bei dem die pharmazeutische Formulation in einer Grunddosierungsform, bei der die optisch aktive Zusammensetzung in einer Menge im Bereich von 0,5 bis 500 mg, zum Beispiel 5 bis 100 mg, z.B. 10 bis 50 mg, pro Dosierungseinheit, vorhanden ist.

**10.** Verfahren oder Verwendung nach einem der Ansprüche 2 bis 9, bei der die Krankheit eine Folge eines Fehlers des zentralen cholinergen Systems ist.

**11.** Verfahren oder Verwendung nach einem der Ansprüche 2 bis 9, bei der die Krankheit Altersdemenz vom Alzheimertyp ist.

**12.** Verfahren oder Verwendung nach Anspruch 11, bei denen die Adminstration:

(i) über den oralen Weg in einer Menge der optisch aktiven Zusammensetzung, die im Bereich von 0,1 bis 60 mg/kg; zum Beispiel 0,5 bis 10 mg/kg, z.B. 1 bis 5 mg/kg Körpergewicht, liegt; oder

(b) über den parenteralen Weg in einer Menge der optische aktiven Zusammensetzung, die im Bereich von 0,01 bis 40 mg/kg; zum Beispiel 0,05 bis 5 mg/kg, z.B. 0,1 bis 2 mg/kg Körpergewicht liegt,

erfolgt.

**Revendications**
**Revendications pour les Etats contractants suivants : DE, GB, FR, IT, NL, SE, LI, CH, BE, AT, LU**

**1.** Composition optiquement active contenant en mélange (a) l'isomère optique individuel (-)-cis-2-méthylspiro(1,3-oxathiolanne-5,3')quinuclidine ou un de ses sels d'addition d'acides ; et (b) l'isomère optique individuel (+)-cis-2-méthylspiro(1,3-oxathiolanne-5,3')quinuclidine ou un de ses sels d'addition d'acides, sous réserve que le racémate (±)-cis soit exclu.

**2.** Composition optiquement active selon la revendication 1, pour l'utilisation en thérapeutique, par exemple dans le traitement des maladies du système nerveux central chez les mammifères.

**3.** Utilisation pour la préparation d'un médicament pour le traitement des maladies du système nerveux central chez les mammifères d'une composition optiquement active selon la revendication 1.

**4.** Formulation pharmaceutique (par exemple sous forme d'une dose unitaire) pour l'utilisation dans le traitement des maladies du système nerveux central chez les mammifères, qui comprend une composition optiquement active selon la revendication 1.

**5.** Utilisation ou formulation selon la revendication 3 ou la revendication 4 où ladite composition contient au moins un ingrédient additionnel (et est par exemple sous une forme en deux parties), l'ingrédient additionnel étant choisi parmi les inhibiteurs de cholinestérase (par exemple la physostigmine, la tétrahydroaminoacridine), les précurseurs de l'acétylcholine (par exemple la choline, la lécithine), les médicaments nootropes (par exemple le piracétam, l'aniracétam, le pramiracétam, l'oxiracétam), les composés qui réagissent avec les canaux $Ca^{++}$ (par exemple la 4-aminopyridine, la 3,4-diaminopyridine), les peptides qui ont des effets modulateurs sur la libération de l'acétylcholine (par exemple la somatostatine), les agents antimuscariniques périphériques (par exemple la pirenzépine, la N-méthylatropine, la N-butylscopolamine), la scopolamine (par exemple la scopolamine transdermique), les agonistes adrénergiques (par exemple la clonidine ou la quanfamicine) et le facteur de croissance du nerf (NGF).

**6.** Utilisation ou formulation selon l'une quelconque des revendications 3, 4 et 5 où ladite composition contient de plus au moins un véhicule ou diluant inertes.

**7.** Formulation pharmaceutique selon l'une quelconque des revendications 4, 5 et 6, qui est sous une forme appropriée à l'administration orale, rectale, parentérale ou transdermique ou pour l'administration par insufflation ou pulvérisation nasale.

**8.** Formulation pharmaceutique selon la revendication 7, qui est sous une forme appropriée à l'administration transdermique et qui comprend, comme composant additionnel, un acide gras de bas poids moléculaire.

**9.** Formulation pharmaceutique selon l'une quelconque des revendications 4 à 8, sous forme d'une dose unitaire, où ladite composition optiquement active est présente en une quantité dans la gamme de 0,5 à 500 mg, par exemple de 5 à 100 mg, telle que 10 à 50 mg par dose unitaire.

13

EP 0 303 391 B1

10. Composition, utilisation ou formulation selon l'une quelconque des revendications 2 à 9, où la maladie est due à une déficience du système cholinergique central.

11. Composition, utilisation ou formulation selon l'une quelconque des revendications 2 à 9, où la maladie est la démence sénile de type Alzheimer.

12. Composition, utilisation ou formulation selon la revendication 11, où l'administration est :
(i) via la voie orale en une quantité de la composition optiquement active qui se situe dans la gamme de 0,1 à 60 mg/kg, par exemple de 0,5 à 10 mg/kg, telle que 1 à 5 mg/kg de poids corporel ; ou
(ii) via la voie parentérale en une quantité de la composition optiquement active qui se situe dans la gamme de 0,01 à 40 mg/kg, par exemple de 0,05 à 5 mg/kg, telle que 0,1 à 2 mg/kg de poids corporel.

**Revendications pour l'Etat contractant suivant : ES**

1. Procédé pour préparer une composition optiquement active contenant en mélange (a) l'isomère optique individuel (-)-cis-2-méthylspiro(1,3-oxathiolanne-5,3')quinuclidine ou un de ses sels d'addition d'acides ; et (b) l'isomère optique individuel (+)-cis-2-méthylspiro(1,3-oxathiolanne-5,3')quinuclidine ou un de ses sels d'addition d'acides, sous réserve que le racémate (±)-cis soit exclu, soit par mélange de (a) et (b), soit par dédoublement optique partiel du racémate (±)-cis.

2. Procédé selon la revendication 1, où la composition optiquement active est destinée à l'utilisation en thérapeutique, par exemple dans le traitement des maladies du système nerveux central chez les mammifères.

3. Utilisation, pour la préparation d'un médicament pour le traitement des maladies du système nerveux central chez les mammifères, d'une composition optiquement active définie dans la revendication 1.

4. Procédé pour la production d'une formulation pharmaceutique (par exemple sous forme d'une dose unitaire) pour l'utilisation dans le traitement des maladies du système nerveux central chez les mammifères, par incorporation à la formulation d'une composition optiquement active définie dans la revendication 1.

5. Utilisation ou procédé selon la revendication 3 ou la revendication 4, où ladite composition contient au moins un ingrédient additionnel (et est par exemple sous une forme en deux parties), l'ingrédient additionnel étant choisi parmi les inhibiteurs de cholinestérase (par exemple la physostigmine, la tétrahydroaminoacridine), les précurseurs de l'acétylcholine (par exemple la choline, la lécithine), les médicaments nootropes (par exemple le piracétam, l'aniracétam, le pramiracétam, l'oxiracétam), les composés qui réagissent avec les canaux $Ca^{++}$ (par exemple la 4-aminopyridine, la 3,4-diaminopyridine), les peptides qui ont des effets modulateurs sur la libération de l'acétylcholine (par exemple la somatostatine), les agents antimuscariniques périphériques (par exemple la pirenzépine, la N-méthylatropine, la N-butylscopolamine), la scopolamine (par exemple la scopolamine transdermique), les agonistes adrénergiques (par exemple la clonidine ou la quanfamicine) et le facteur de croissance du nerf (NGF).

6. Utilisation ou procédé selon l'une quelconque des revendications 3, 4 et 5, où ladite composition contient de plus au moins un véhicule ou diluant inertes.

7. Procédé selon l'une quelconque des revendications 4, 5 et 6, où la formulation pharmaceutique est sous une forme appropriée à l'administration orale, rectale, parentérale ou transdermique ou pour l'administration par insufflation ou pulvérisation nasale.

8. Procédé selon la revendication 7, où la formulation pharmaceutique est sous une forme appropriée à l'administration transdermique et qui comprend, comme composant additionnel, un acide gras de bas poids moléculaire.

14

**9.** Procédé selon l'une quelconque des revendications 4 à 8, où la formulation pharmaceutique est sous forme d'une dose unitaire, et ladite composition optiquement active est présente en une quantité dans la gamme de 0,5 à 500 mg, par exemple de 5 à 100 mg, telle que 10 à 50 mg par dose unitaire.

**10.** Procédé ou utilisation selon l'une quelconque des revendications 2 à 9, où la maladie est due à une déficience du système cholinergique central.

**11.** Procédé ou utilisation selon l'une quelconque des revendications 2 à 9, où la maladie est la démence sénile de type Alzheimer.

**12.** Procédé ou utilisation selon la revendication 11, où l'administration est :

(i) via la voie orale en une quantité de la composition optiquement active qui se situe dans la gamme de 0,1 à 60 mg/kg, par exemple de 0,5 à 10 mg/kg, telle que 1 à 5 mg/kg de poids corporel ; ou

(ii) via la voie parentérale en une quantité de la composition optiquement active qui se situe dans la gamme de 0,01 à 40 mg/kg, par exemple de 0,05 à 5 mg/kg, telle que 0,1 à 2 mg/kg de poids corporel.